# EUROPEAN PATENT SPECIFICATION

(11) **EP 1 624 762 B1**
(45) Date of publication and mention of the grant of the patent: **28.02.2007**
(21) Application number: 04733714.2
(22) Date of filing: 18.05.2004
(51) Int. Cl.: A23C 9/127, A61K 35/74, A23L 1/03, A61P 1/00

(54) **A PROBIOTIC COMPOSITION COMPRISING AT LEAST TWO LACTIC ACID BACTERIAL STRAINS WHICH ARE ABLE TO COLONISE THE GASTROINTESTINAL TRACTS IN COMBINATION WITH HAVING INTESTINAL SURVIVAL PROPERTY, INTESTINAL BINDING PROPERTY, AN INFECTION PROTECTION PROPERTY AND A FIBER FERMENTING PROPERTY**
PROBIOTISCHE ZUSAMMENSETZUNG MIT MINDESTENS ZWEI MILCHSÄUREBAKTERIENSTÄMMEN, DIE ZUR KOLONISIERUNG DES MAGEN-DARM-TRAKTS FÄHIG SIND UND IN KOMBINATION DAMIT DIE EIGENSCHAFTEN, IM DARM ZU ÜBERLEBEN, AN DEN DARM ZU BINDEN, GEGEN INFEKTION ZU SCHÜTZEN UND BALLASTSTOFFE ZU FERMENTIEREN, AUFWEISEN
COMPOSITION PROBIOTIQUE COMPRENANT AU MOINS DEUX SOUCHES BACTERIENNES D'ACIDE LACTIQUE CAPABLES DE COLONISER LES TRACTUS GASTRO-INTESTINAUX ET DE POSSEDER EN MEME TEMPS UNE PROPRIETE DE SURVIE INTESTINALE, UNE PROPRIETE DE LIAISON INTESTINALE, UNE PROPRIETE DE PROTECTION CONTRE LES INFECTIONS.

(30) Priority: 22.05.2003 SE 0301516
(43) Date of publication of application: 15.02.2006
(73) Proprietor: Synbiotics AB, 105 46 Stockholm (SE)
(72) Inventor: LJUNGH-WADSTRÖM, Asa, S-224 67 Lund (SE); WADSTRÖM, Torkel, S-224 67 Lund (SE); BENGMARK, Stig, S-263 32 Höganäs (SE)
(74) Representative: Ström, Tore
(86) International application number: PCT/SE2004/000796
(87) International publication number: WO 2004/103083

(56) References cited:
- WO-A1-97/29645
- WO-A1-99/29833
- WO-A2-01/58465
- US-A- 6 022 568
- US-A1- 2002 022 019
- US-B1- 6 410 305
- ROLFE R.D.: 'The role of probiotic cultures in the control of gastrointestinal health' THE JOURNAL OF NUTRITION vol. 130, 2000, pages 396S - 402S, XP000978812 & DATABASE MEDLINE (NLM) [Online] Database accession no. 10721914

## Description

The invention relates to a new probiotic composition. More precisely, the invention refers to a probiotic composition comprising at least two lactic acid bacterial strains having at least two significant properties for the maintenance of the intestinal microbial ecosystem, for the prevention and treatment of gastrointestinal disturbances, and for colonizing gastrointestinal tracts.

The enteric flora comprises approximately 95% of the total number of cells in the human body. The importance of the intestinal microflora and, more specifically its composition, in physiological as well as pathophysiological processes in the gastrointestinal tract of adult humans has become more and more evident.

Health effects related to changes in the intestinal microflora have been attributed to viable microorganisms (bacteria or yeast) that have a beneficial effect on the health of the host. The presence of lactic acid bacteria has been found to be significant for the maintenance of the intestinal microbial ecosystem. These microorganisms, since long called probiotics, are commonly defined as viable microorganisms that exhibit a beneficial effect on the health of the host when they are ingested. Thus, a probiotic can be defined as a viable monoculture or a mixed culture of microorganisms, which affects the host by improving the properties of indigenous microflora in the gastrointestinal tract. Presently, a number of commercial products are available for the prevention and treatment of multiple gastrointestinal disturbances.

In EP 1 020 123 A1 beverages in combination with a mixture of lyophilized live lactic bacteria in a non-milk matrix are described. The mixture of lyophilized live lactic bacteria comprises at least three of *Brevibacterium breve, B. infantis, B. longum, B. bifidum, Lactobacillus acidophilus, L. bulgaricus, L. casei, L. plantarum, Streptococcus thermophilus* and *Streptococcus faecium.* The beverages are intended to supplement and balance the intestinal flora as well as supply other beneficial supplements, such as vitamins and antioxidants, to the consumer.

However, the literature contains many conflicting observations for their proposed benefits, and the corresponding mechanism of action is many times undefined.

Possibly successful probiotic strains have been traditionally incorporated into fermented milk products. In the case of novel microorganisms and modified organisms the to benefit ratio between the question of their safety and the risk of ingestion has to be assessed. Lactic acid bacteria in foods have a long history of safe use.

During the last few years these organisms have been included in functional foods and health-related products. The definition for probiotics has gradually changed with increasing understanding of the mechanisms by which they influence human health. While the health claims are generally accepted by both scientists and consumers, the underlying molecular mechanisms of many of the claimed probiotic properties still remain controversial.

Lactic acid bacteria have successfully been isolated and identified, which exhibit beneficial probiotic traits. These characteristics include the demonstration of bile tolerance, acid resistance, adherence to host epithelial tissue, and in vitro antagonism of potentially pathogenic microorganisms, or those which have been implicated in promoting inflammation.

On the market, there exist several milk and fruit based products containing lactic acid bacteria. The specific interactions with the gastrointestinal tract are often sparsely described, and the doses are poorly defined.

The probiotic microorganisms must be able to be manufactured under industrial conditions. Furthermore, they have to survive and retain their functionality during storage as well as in the foods, into which they are to be incorporated without producing negative effects. Studies have shown low viability of probiotics in market preparations.

For the administration of lactic acid bacteria as probiotics in humans or animals, they should be adapted to the specific conditions of the gastrointestinal tract of adults, which is significantly different from that of new-borns. However, up to now there exist poor evidence of a successful implanting of a given strain into the dominant flora of a healthy adult individual. Such an implantation can succeed only at the moment of birth or when the probiotic organism is administered to a patient having an extremely unbalanced intestinal microflora, for example after prolonged antibiotic treatment.

Various producers of various probiotics do often claim health benefits from supplying different probiotics. In most cases this is unsubstantiated and not true. The reality is that only a small minority of the lactic acid bacteria have the health potentials requested. Most of the probiotics on the market do not survive the acidity of the stomach or the bile acid content of the small intestine, nor do they adhere to the colonic mucosa and even temporary colonize the stomach.

The purpose of the invention is to avoid the above-mentioned drawbacks according to the state of the art by providing a probiotic composition having an optimal capacity to survive and colonize the gastrointestinal tracts of not only humans but also of those animals which have a similar digestive tract and thus a corresponding intestinal microflora.

In order to achieve this purpose the method according to the invention has obtained the characterizing features of claim 1.

The composition according to the invention is based on that two or more lactic acid bacteria (LAB) strains of different species jointly should have properties that should be beneficial for human intake while simulating the effect of an implanted flora. By having at least two properties together, which define well-established criteria for gastrointestinal survival and/or colonization, the potential health benefits and influence on the gut flora is increased. According to the invention, new probiotic LAB-strains of active beneficial organisms are provided, which have specific favourable functional characteristics.

LAB-strains of different species for the composition according to the invention were isolated from the human large intestinal mucosa of deceased persons who died from other causes than diseases in the GI tract, and whom had not been treated by antibiotics during at least two months prior to death (F-strains and 2362). The other strains were isolated from fermented rye.

The LAB-strains of different species were typed to the species level by API 50CH as well as ribotyping (the Swedish Institute for Food and Biotechnology).

The specific strains to be used in the probiotic composition according to the invention are *Lactobacillus plantarum* F5, *Lactobacillus plantarum* F26, *Lactobacillus plantarum* 2592, *Lactobacillus paracasei* (*paracasei*) F19, *Pediococcus penosaceus* 16:1, *Lactobacillus plantarum* 50:1, and *Leuconostoc mesenteroides* 77:1.

The bacterial strains were deposited on June 19, 2001 pursuant to, and in satisfaction of, the requirements of the Budapest Treaty on the International Recognition of the Deposit of Microorganisms for the Purposes of Patent Procedure with the Belgian Coordinated Collection of Microorganisms (BCCM), Gent, Belgium, under Accession No. LMG P-2060,4 for *Lactobacillus plantarum* F5, Accession No. LMG P-20605 for *Lactobacillus plantarum* F26, Accession No. LMG P-20606 for *Lactobacillus plantarum* 2592, Accession No. LMG P-20607 for *Leuconostoc mesentorides* 77:1, and Accession No. LMG P-20608 for *Pediococcus penosaceus* 16:1.

*Lactobacillus plantarum* 50:1 was deposited with the Belgian Coordinated Collection of Microorganisms, on June 21, 2001, where it obtained the Accession No. P-20609.

*Lactobacillus paracasei* (*paracasei*) F19 has earlier been deposited with the Belgian Coordinated Collection of Microorganisms, where it obtained the Accession No. LMG P-17806.

All the LAB-strains of different species were grown aerobically on MRS agar at 37°C for 24 hrs. In a further experiment, growth at different temperatures was determined. All strains could multiply at temperatures from +4°C to 40°C or 45°C. All strains produced protease(s) as determined by skim milk agar assay (24 hrs, aerobically, 37°C). No strain produced nitrite or nitrate.

All the LAB-strains of different species could utilize prebiotics, such as inulin and amylopectin, but not β-glucan as determined by growth on YNB medium with such fibers as a sole carbon source. Since the strains ferment these fibers, they should exert a beneficial effect in the colonic flora. An enhanced fiber degradation also increases crude fibre digestibility.

In a composition according to the invention a mixture of cocci and bacilli should be optimal since cocci and bacilli have different generation times, bacilli proliferating much faster than cocci. An optimal growth is accomplished by preferably including at least one lactic acid bacillus strain and at least one lactic acid coccus strain in the composition.

In order promote growth of the lactic acid bacterial strains and succeed it the colonization of the epithelium of the gastrointestinal tract of a host as well as exerting a resistance to infectious diseases a composition according to the invention should have an intestinal survival property, an intestinal binding property, an infection protecting property, and a fiber fermenting property.

A significant intestinal survival property is the ability to grow in the presence of bile. All the claimed LAB-strains are able to grow in the presence of 20% bile (human and porcine) and then retain their bile-tolerance after the selective pressure has been removed and reapplied.

All the LAB-strains of different species do also survive when they are subjected to an acidity of pH 2.0-3.0. Furthermore, this acid resistance remains with the addition of 0.3 % pepsin (24 hrs at 37°C) in three of them (*L. plantarum* F5, *L. plantarum* F26, and *L. plantarum* 50:1) as depicted in Table 1 below.

**Table 1**

| Strain | pH | pepsin |
|---|---|---|
| *L. plantarum* F5 | 2.0 | + |
| *L. paracasei* (*parac.*) F19 | 2.5 | |
| *L. plantarum* F26 | 2.0 | + |
| *L. plantarum* 2592 | 2.5 | |
| *P. pentosaceus* 16:1 | 2.5 | |
| *L. plantarum* 50:1 | 2.0 | + |
| *L. mesenteroides* 77:1 | 3.0 | |

Thus, the acid and bile tolerant strains possess growth advantages over that of the parent strains under stress conditions.

BalbC mice were fed the inventive lactic acid bacteria intragastrically. The LAB-strains used were *L. paracasei* (*paracasei*) F19, *L. plantarum* 2592, *P. pentosaceus* 16:1, and *L. mesenteroides* 77:1. The excretion of the four strains given was obtained by culturing. Excretion of these could be detected during 6 weeks after the administration, which indicates colonization of the gastrointestinal tract of the host.

The acid response of these LAB-strains, when exposed to sublethal adaptive acid conditions (pH 5.0 for 60 min), was found to confer a significant level of protection against subsequent exposure to lethal pH (pH 3.0) as well as to different environmental stresses (oxidative stress, ethanol exposure and freezing).

An acid tolerant response developed during adaptation at pH 5.0 affected the cell survival against environmental stresses. Adapted cultures developed tolerance to ethanol (20%), freezing (-20°C), and oxidative challenge (10 mM H₂O₂) but not to heating (60°C) and osmotic shock (3 M NaCl). The presence of chloramphenicol during the adaptation step partially inhibited the cell resistance. This adaptation was found to be dependent on a *de novo* protein synthesis.

An exposure to acid stress at pH 5.0 for 1 h caused the induction of nine new proteins with molecular weights (MW) from 10.1 to 68.1 kDa as determined by sodium dodecyl sulphate polyacrylamide gel electrophoresis, whereby the over-expression of the proteins also could be established. All other proteins were repressed, which exhibited basic or neutral characteristics.

*Lactobacillus plantarum* 2592 produces large amounts of a characteristic protein having a molecular weight of 19 kDa.

The proteins induced during the acid tolerance response have to be active since the incorporation of amino acid analogues inhibited the response.

Several of the induced and over-expressed proteins were also found to cross-react with earlier described heat-shock proteins. Proteins of molecular weight 10, 24 and 43 kDa cross-reacted with cochaperons GroES, GrpE and DnaJ, respectively. Less over-expressed proteins of molecular weight 70 and 55 kDa cross-reacted with GrpE and GroES, respectively.

Thus, the survival under acid stress conditions was found to be linked to the expression of an adaptive stress response. Such a response, characterized by the transient induction of specific proteins and physiological changes, enhances the ability of the LAB-strains of different species to withstand harsh environmental conditions. The continued protein synthesis of the specific proteins induced by the claimed LAB-strains in an acid environment, like in the gastric stomach, would then increase the stability of preexisting proteins.

A further infection protecting property of the LAB-strains of different species is their antioxidant properties, whereby the action of free radicals, by products of inflammation, are counteracted.

The LAB-strains produced antioxidants as measured by a spectrophotometric assay (Total Antioxidant kit, product no. NX 2332 from Randox, San Diego, CA, USA.) in lysates of lactic acid bacterial strains. It was shown that antioxidants are produced, which are effective against free radicals and terminate oxidative chain reactions. All the LAB-strains produced antioxidants in amounts from 2.7 to 8.9 mg protein per lit. The strains *P. pentosaceus* 16:1 and *L. plantarum* F26 produced the largest amounts of antioxidants immediately followed by *L. paracasei* (*paracasei*) F19.

Diabetic mice were given these LAB-strains in the same dose daily for 12 days. The mice were fed 10¹⁰ cells of four strains (*L. paracasei* (*paracasei*) F19, *L. plantarum* 2592, *P. pentosaceus* 16:1, and *L. mesenteroides* 77:1) twice daily of these strains intragastrically for 12 days. The cholesterol levels of the animals were not decreased. The safety of the strains was confirmed by taking blood cultures of the mice at the end of the study, which showed no growth.

Likewise, 52 healthy persons (14-87 years of age) consumed 10¹⁰ bacteria of 4 of the LAB-strains of different species daily for 3 months without adverse effects.

Preferably, the infection protecting property of the inventive composition is an immunopotentiating effect, whereby a positive immune response is obtained. The strains were found to have different abilities of transcribing NF-kappa B to the cell nucleus as determined by a dot blot (Wilson L, et al., Gastroenterol 1999; 117:106-114; and Splecker M, et al., J Immunol 2000;15 (March):3316-22). The induction of NF-kappa B resulted in a cytokin response, which either was pro-inflammatory or anti-inflammatory.

The *Lactobacillus* strains, particularly *L. paracasei* (*paracasei*) F19 but not the cocci, transcribed NF-kappa B in the macrophage cell line U973, resulting in the synthesis of the interleukins IL-1β and IL-8. The cytokines induced were of the pro-inflammatory type.

A further important aspect of the composition according to the invention is that the probiotic LAB-strains according to the invention therein should have an intestinal binding property in order to be able to developing their functional properties. One such binding property is that they shall exhibit high adhesion to intestinal tracts. To act as a probiotic, a lactic acid bacterial strain must be able to colonize the intestinal mucus layer.

The claimed strains were also obtained by screening for binding of porcine mucin (type II, Sigma Chem Co, St Louis, CO, USA)) in an ELISA method (Tuomola EM, *et al.,* FEMS Immunology and Med Microbiol 26(1999) 137-142). The results are shown in Table 2 below. The results were compared with those of a commercial strain, *Lactobacillus rahmnosus* GG (Valio).

**Table 2**

| Strain | Mucin binding | SAT |
|---|---|---|
| *L. plantarum* F5 | 0.557 | 1M |
| *L. para casei* (*parac*) F19 | 0.515 | 0.1M |
| *L. plantarum* F26 | 0.582 | 1M |
| *L. plantarum* 2592 | 0.707 | 1M |
| *P. pentosaceus* 16:1 | 1.052 | <0.1M |
| *L. plantarum* 50:1 | 0.883 | 2M |
| *L. mesenteroides* 77:1 | 1.265 | <0.1M |
| *L. rahmnosus* GG | 0.2 | 4M |

Apart from exhibiting mucin binding, the probiotic LAB-strains in the inventive composition should be able to express cell surface hydrophobicity in the human gastrointestinal tract. The cell surface hydrophobicity was measured by the Salt Aggregation Test (SAT) (Rozgoynyi F, *et al.*, FEMS Microbiol Lett 20(1985) 131-138). In Table 1 below a comparison is shown for the adhesiveness of vibronectin (Vn), vibronectin at pH 3.5, fetuin (Ft), asialofetuin, and asialofetuin at pH 3.5.

**Table 3**

| | Vn | | | asialo- | asialo- |
|---|---|---|---|---|---|
| Strain | Vn | pH 3.5 | Ft* | Ft | Ft pH 3.5 |
| *L. plantarum* F5 | ++ | +++ | - | + | +++ |
| *L. paracasei* F19 | +++ | +++ | +++ | +++ | +++ |
| *L. plantarum* F26 | + | +++ | + | +++ | + |
| *L. plantarum* 2592 | - | + | + | +++ | - |
| *P. pentosaceus* 16:1 | +++ | + | - | + | - |
| *L. plantarum* 50:1 | +++ | + | - | ++ | + |
| *L. mesenteroides* 77:1 | - | ++ | - | + | + |

| | | | | | |
|---|---|---|---|---|---|
| * denotes identical results at pH 7 and pH 3.5 | | | | | |

Adhered microorganisms were found to be tightly bound to the immobilized mucus. Five strains expressed a pronounced cell surface hydrophobicity, and the other three strains expressed moderate cell surface hydrophobicity. No correlation was observed between cell surface hydrophobicity and the adhesive ability of the LAB-strains.

The LAB-strains of different species were also tested for binding to bovine submaxillary glands and porcine type II mucin (Sigma) in a particle agglutination assay (PAA) (Paulsson M, *et al.,* J Clin Microbiol 30(1992) 2006-20012). Generally, expression of binding to bovine mucus was found to be stronger.

Since many of the cells in tissues of multicellular organisms are embedded in an extracellular matrix consisting of secreted proteins and polysaccharides, the LAB-strains of different species according to the invention were examined with reference to their interaction with gastrointestinal extracellular matrix protein. The binding of extracellular matrix proteins and glucosaminoglycans was studied in the above mentioned immobilized form (PAA assay). All the LAB-strains expressed binding to collagen type I and III, fibronectin, fibrinogen, and heparin, also at pH 3.5. The strains *L. paracasei* (*paracasei*) F19, *L. plantarum* F26, and *L. plantarum* 2592 expressed binding of fetuin, also at pH 3.5.

Furthermore, all the LAB-strains of different species, except *L. plantarum* 2592 and *L. mesenteroides* 77:1, expressed binding to vitronectin. However, at pH 3.5, these strains expressed a weak binding.

A further significant intestinal survival property of the LAB-strains in the probiotic composition according to the invention is to have an infection protecting property.

*Heliobacter pylori* is the causative agent of acute and chronic gastritis, one of the most prevalent infections world-wide which may proceed to atrophic gastritis, adenocarcinoma and MALT lymphoma.

The lactobacilli of the present invention do inhibit the growth of 10 strains of *H. pylori* due to the concentration of lactic acid and the pH *in vitro.* The inhibitory effect was lost when the pH was adjusted to 6.0. Further analyses showed that L-lactic acid, but not D-lactic acid or acetic acid, inhibited growth at concentrations of 60 to 100 mM. No relation between the CagA phenotype of *H. pylori* and the tolerance to lactic acid was observed. The inhibition was found to be strain-specific.

A further infection protecting property is manifested by the interactions mediated by bacteriocins. All the LAB-strains of different species, except *L. plantarum* F5, secrete into a cell-free supernatant a product having antimicrobial activity. The products produced were heat-stable antimicrobial compounds, which were shown to be proteineous in nature and, therefore, referred to as a bacteriocins. The bacteriocins exhibited activities against grampositive organisms. Some were active against gramnegative organisms (*H. pylori* strains), and some against yeasts (Candida strains). The antimicrobial effects were not directly correlated to the production of acid.

Furthermore, *H. pylori* cells were bacillary in their shape and not coccoid, indicating that the observed inhibition was related to a bactericidal effect rather than an induction of viable but non-culturable coccoid forms. The bactericidal effect was found to be due to an intracellular 28 kDa protein, which was released after lysis. Proteolytic treatment of this intracellular protein resulted in loss of antibacterial activity. This loss could be abolished by re-naturing by means of sodium dodecyl sulphate polyacrylamide gel electrophoresis.

In an established model of *H. pylori* gastritis in mice the administration of the LAB-strains of different species according to the invention resulted in an inhibited infection with an accompanied decreased inflammation. This inhibitory effect appeared to be strain-specific rather than species-specific. The in vitro activity of the LAB-strains correlated well with activity against *H. pylori.*

Preparations of the probiotic composition according to the invention comprise chilled, frozen, or lyophilized live bacteria of at least 10¹⁰ CFU/g as a probiotic additive in food or feed.

## Claims

1. A probiotic composition comprising at least two lactic acid bacterial strains, **characterized in that** said at least two lactic acid bacterial strains are able colonize the gastrointestinal tract of humans and animals and in combination have at least two beneficial properties, which are an intestinal survival property, an intestinal binding property, an infection protecting property, and a fiber fermenting property, said at least two lactic acid bacterial strains being selected from the group comprising *Lactobacillus plantarum* F5 (LMG P-20604), *Lactobacillus plantarum* F26 (LMG P-20605), *Lactobacillus plantarum* 2592 (LMG P-20606), *Pediococcus penosaceus* 16:1 (LMG P-20608), and *Leuconostoc mesentorides* 77:1 (LMG P-20607), *Lactobacillus plantarum* 50:1 (P-20609), and *Lactobacillus paracasei* (*paracasei*) F19 (LMG P-17806).

2. A probiotic composition as in claim 1, **characterized in that** said lactic acid bacterial strains are viable bacteria of at least 10¹⁰ CFU/g.

3. A probiotic composition as in claim 1, **characterized in that** said intestinal survival property is ability to grow in the presence of bile.

4. A probiotic composition as in claim 1, **characterized in that** said intestinal survival property is ability to survive at a low pH.

5. A probiotic composition as in claim 4, **characterized in that** said ability to survive at low pH is survival at low pH in the presence pepsin.

6. A probiotic composition as in claim 1 and 4, **characterized in that** said intestinal survival property is ability to produce stress proteins.

7. A probiotic composition as in claim 6, **characterized in that** said stress proteins cross-react with heat shock proteins.

8. A probiotic composition as in claim 1, **charaterized in that** said intestinal binding property is ability to bind to mucin.

9. A probiotic composition as in claim 1, **characterized in that** said intestinal binding property is ability to bind to extracellular matrix proteins.

10. A probiotic composition as in claim 1, **characterized in that** said intestinal binding property is ability to bind to glucosaminoglycans.

11. A probiotic composition as in claim 1, **characterized in that** said intestinal binding property is ability to express cell surface hydrophobicity.

12. A probiotic composition as in claim 1, **characterized in that** said infection protecting property is ability to produce bacteriocins.

13. A probiotic composition as in claim 12, **characterized in that** said bacteriocins have activity against grampositive bacteria.

14. A probiotic composition as in claim 12, **characterized in that** said bacteriocins have activity against gramnegative bacteria.

15. A probiotic composition as in claim 12, **characterized in that** said bacteriocins have activity against yeast.

16. A probiotic composition as in claim 1, **characterized in that** said infection protecting property is ability to produce antioxidants.

17. A probiotic composition as in claim 1, **characterized in that** said infection protecting property is ability to induce a pro-inflammatory cytokin response.

18. A probiotic composition as in claim 1, **characterized in that** said fiber fermenting property is ability to ferment amylopectin and inulin.

19. Use of a lactic acid bacterial strain, selected from the group comprising *Lactobacillus plantarum* F5 (LMG P-20604), *Lactobacillus plantarum* F26 (LMG P-20605), *Lactobacillus plantarum* 2592 (LMG P-20606), *Pediococcus penosaceus* 16:1 (LMG P-20608), and *Leuconostoc mesentorides* 77:1 (LMG P-20607), and *Lactobacillus plantarum* 50:1 (P-20609), alone or in combination, as a probiotic additive in the preparation of food or feed.

## Patentansprüche

1. Probiotische Zusammensetzung, die mindestens zwei Milchsäurebakterienstämme umfasst,
**dadurch gekennzeichnet, dass**
die mindestens zwei Milchsäurebakterienstämme zur Besiedelung des Gastrointestinaltrakts von Menschen und Tieren fähig sind und in Kombination mindestens zwei zuträgliche Eigenschaften aufweisen, wobei es sich um eine Eigenschaft im Darm zu überleben, eine Eigenschaft an den Darm zu binden, eine Eigenschaft gegen Infektion zu schützen und eine Eigenschaft Ballaststoffe zu fermentieren handelt, die mindestens zwei Milchsäurebakterienstämme aus der Gruppe ausgewählt werden, die *Lactobacillus plantarum* F5 (LMG P-20604), *Lactobacillus plantarum* F26 (LMG P-20605), *Lactobacillus plantarum* 2592 (LMG P-20606), *Pediococcus penosaceus* 16:1 (LMG P-20608) und *Leuconostoc mesentorides* 77:1 (LMG P-20607), *Lactobacillus plantarum* 50:1 (P-20609), und *Lactobacillus paracasei (paracasei)* F19 (LMG P-17806) enthält.

2. Probiotische Zusammensetzung nach Anspruch 1,
**dadurch gekennzeichnet, dass**
die Milchsäurebakterienstämme lebensfähige Bakterien von mindestens 10¹⁰ CFU/g sind.

3. Probiotische Zusammensetzung nach Anspruch 1,
**dadurch gekennzeichnet, dass**
die Eigenschaft im Darm zu überleben die Fähigkeit ist, in der Gegenwart von Galle zu wachsen.

4. Probiotische Zusammensetzung nach Anspruch 1,
**dadurch gekennzeichnet, dass**
die Eigenschaft im Darm zu überleben die Fähigkeit ist, bei einem niedrigen pH-Wert zu überleben.

5. Probiotische Zusammensetzung nach Anspruch 4,
**dadurch gekennzeichnet, dass**
die Fähigkeit des Überlebens bei einem niedrigen pH-Wert das Überleben bei einem niedrigen pH-Wert in Gegenwart von Pepsin ist.

6. Probiotische Zusammensetzung nach den Ansprüchen 1 und 4,
**dadurch gekennzeichnet, dass**
die Eigenschaft im Darm zu überleben die Fähigkeit ist, Stressproteine zu produzieren.

7. Probiotische Zusammensetzung nach Anspruch 6,
**dadurch gekennzeichnet, dass**
die Stressproteine mit Hitzeschockproteinen kreuzreagieren.

8. Probiotische Zusammensetzung nach Anspruch 1,
**dadurch gekennzeichnet, dass**
die Eigenschaft an den Darm zu binden die Fähigkeit ist, an Muzin zu binden.

9. Probiotische Zusammensetzung nach Anspruch 1
**dadurch gekennzeichnet, dass**
die Eigenschaft an den Darm zu binden die Fähigkeit ist, an extrazelluläre Matrixproteine zu binden.

10. Probiotische Zusammensetzung nach Anspruch 1,
**dadurch gekennzeichnet, dass**
die Eigenschaft an den Darm zu binden die Fähigkeit ist, an Glykosaminoglykane zu binden.

11. Probiotische Zusammensetzung nach Anspruch 1,
**dadurch gekennzeichnet, dass**
die Eigenschaft an den Darm zu binden die Fähigkeit ist, eine Zelloberflächen-Hydrophobizität zu zeigen.

12. Probiotische Zusammensetzung nach Anspruch 1,
**dadurch gekennzeichnet, dass**
die Eigenschaft gegen Infektion zu schützen die Fähigkeit ist, Bakteriozine zu produzieren.

13. Probiotische Zusammensetzung nach Anspruch 12,
**dadurch gekennzeichnet, dass**
die Bakteriozine gegen grampositive Bakterien aktiv sind.

14. Probiotische Zusammensetzung nach Anspruch 12,
**dadurch gekennzeichnet, dass**
die Bakteriozine gegen gramnegative Bakterien aktiv sind.

15. Probiotische Zusammensetzung nach Anspruch 12,
**dadurch gekennzeichnet, dass**
die Bakteriozine gegen Hefen aktiv sind.

16. Probiotische Zusammensetzung nach Anspruch 1,
**dadurch gekennzeichnet, dass**
die Eigenschaft gegen Infektion zu schützen die Fähigkeit ist, Antioxidantien zu produzieren.

17. Probiotische Zusammensetzung nach Anspruch 1,
**dadurch gekennzeichnet, dass**
die Eigenschaft gegen Infektion zu schützen die Fähigkeit ist, eine proinflammatorische Cyotokin-Reaktion zu induzieren.

18. Probiotische Zusammensetzung nach Anspruch 1,
**dadurch gekennzeichnet, dass**
die Eigenschaft Ballaststoffe zu fermentieren die Fähigkeit ist, Amylopektin und Inulin zu fermentieren.

19. Verwendung eines Milchsäurebakterienstammes, der aus der Gruppe ausgewählt wird, die *Lactobacillus plantarum* F5 (LMG P-20604), *Lactobacillus plantarum* F26 (LMG P-20605), *Lactobacillus plantarum* 2592 (LMG P-20606), *Pediococcus penosaceus* 16:1 (LMG P-20608) und *Leuconostoc mesentorides* 77:1 (LMG P-20607) sowie *Lactobacillus plantarum* 50:1 (P-20609) umfasst, alleine oder kombiniert, als ein probiotischer Zusatz bei der Zubereitung von Nahrungs- oder Futtermitteln.

## Revendications

1. Composition probiotique comprenant au moins deux souches bactériennes d'acide lactique, **caractérisée en ce que** lesdites au moins deux souches bactériennes d'acide lactique peuvent coloniser les tractus gastro-intestinaux chez les êtres humains et les animaux et en association avoir au moins deux propriétés bénéfiques, qui sont une propriété de survie intestinale, une propriété de liaison intestinale, une propriété de protection contre les infections et une propriété de fermentation des fibres, lesdites au moins deux souches bactériennes d'acide lactique étant sélectionnées parmi le groupe comprenant *Lactobacillus plantarum* F5 (LMG P-20604), *Lactobacillus plantarum* F26 (LMG P-20605), *Lactobacillus plantarum* 2592 (LMG P-20606), *Pediococcus penosaceus* 16:1 (LMG P-20608) et *Leuconostoc mesentorides* 77:1 (LMG P-20607), *Lactobacillus plantarum* 50:1 (P-20609) et *Lactobacillus paracasei* (*paracasei*) F19 (LMG P-17806).

2. Composition probiotique selon la revendication 1, **caractérisée en ce que** lesdites souches bactériennes d'acide lactique sont des bactéries viables d'au moins 10¹⁰ CFU/g.

3. Composition probiotique selon la revendication 1, **caractérisée en ce que** ladite propriété de survie intestinale est une capacité à croître en présence de bile.

4. Composition probiotique selon la revendication 1, **caractérisée en ce que** ladite propriété de survie intestinale est une capacité à survivre à un pH faible.

5. Composition probiotique selon la revendication 4, **caractérisée en ce que** ladite capacité à survivre à un pH faible est une capacité à survivre à un pH faible en présence de pepsine.

6. Composition probiotique selon les revendications 1 et 4, **caractérisée en ce que** ladite propriété de survie intestinale est une capacité à produire des protéines de stress.

7. Composition probiotique selon la revendication 6, **caractérisée en ce que** lesdites protéines de stress ont une réaction croisée avec les protéines de choc thermique.

8. Composition probiotique selon la revendication 1, **caractérisée en ce que** ladite propriété de liaison intestinale est la capacité à se lier avec la mucine.

9. Composition probiotique selon la revendication 1, **caractérisée en ce que** ladite propriété de liaison intestinale est la capacité à se lier avec des protéines à matrice extracellulaire.

10. Composition probiotique selon la revendication 1, **caractérisée en ce que** ladite propriété de liaison intestinale est la capacité à se lier avec des glucosaminoglycanes.

11. Composition probiotique selon la revendication 1, **caractérisée en ce que** ladite propriété de liaison intestinale est la capacité à exprimer une hydrophobicité de la surface cellulaire.

12. Composition probiotique selon la revendication 1, **caractérisée en ce que** ladite propriété de protection contre les infections est une capacité à produire des bactériocines.

13. Composition probiotique selon la revendication 12, **caractérisée en ce que** lesdites bactériocines ont une activité contre les bactéries Gram-positives.

14. Composition probiotique selon la revendication 12, **caractérisée en ce que** lesdites bactériocines ont une activité contre les bactéries Gram-négatives.

15. Composition probiotique selon la revendication 12, **caractérisée en ce que** lesdites bactériocines ont une activité contre les levures.

16. Composition probiotique selon la revendication 1, **caractérisée en ce que** ladite propriété de protection contre les infections est une capacité à produire des anti-oxydants.

17. Composition probiotique selon la revendication 1, **caractérisée en ce que** ladite propriété de protection contre les infections est une capacité à induire une réaction pro-inflammatoire à la cytokine.

18. Composition probiotique selon la revendication 1, **caractérisée en ce que** ladite propriété de fermentation des fibres est la capacité à fermenter l'amylopectine et l'inuline.

19. Utilisation d'une souche bactérienne d'acide lactique, sélectionnée parmi le groupe comprenant *Lactobacillus plantarum* F5 (LMG P-20604), *Lactobacillus plantarum* F26 (LMG P-20605), *Lactobacillus plantarum* 2592 (LMG P-20606), *Pediococcus penosaceus* 16:1 (LMG P-20608) et *Leuconostoc mesentorides* 77:1 (LMG P-20607), et *Lactobacillus plantarum* 50:1 (P-20609), seuls ou en combinaison, comme additif probiotique dans la préparation d'aliments ou de nourriture pour les animaux.
